(19) Europäisches Patentamt
European Patent Office
Office européen des brevets

(11) **EP 2 598 186 B1**

(12) **EUROPÄISCHE PATENTSCHRIFT**

(45) Veröffentlichungstag und Bekanntmachung des Hinweises auf die Patenterteilung:
**16.03.2016  Patentblatt 2016/11**

(21) Anmeldenummer: **11746170.7**

(22) Anmeldetag: **29.07.2011**

(51) Int Cl.:
**A61M 1/16** (2006.01)

(86) Internationale Anmeldenummer:
**PCT/EP2011/003832**

(87) Internationale Veröffentlichungsnummer:
**WO 2012/016671 (09.02.2012 Gazette 2012/06)**

(54) **VORRICHTUNG UND VERFAHREN ZUR ERKENNUNG DER RICHTUNG DER FLÜSSIGKEITSSTRÖMUNG DURCH EINEN DIALYSATOR**

DEVICE AND METHOD FOR DETECTING THE DIRECTION OF THE FLOW OF LIQUID THROUGH A DIALYSER

DISPOSITIF ET PROCÉDÉ D'IDENTIFICATION DU SENS DE L'ÉCOULEMENT D'UN LIQUIDE À TRAVERS UN DIALYSEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.07.2010   DE 102010032980**

(43) Veröffentlichungstag der Anmeldung:
**05.06.2013   Patentblatt 2013/23**

(73) Patentinhaber: **Fresenius Medical Care Deutschland GmbH**
**61352 Bad Homburg v.d.H. (DE)**

(72) Erfinder: **KOPPERSCHMIDT, Pascal**
**97456 Dittelbrunn (DE)**

(74) Vertreter: **Oppermann, Frank**
**OANDO Oppermann & Oppermann LLP**
**Washingtonstrasse 75**
**65189 Wiesbaden (DE)**

(56) Entgegenhaltungen:
**EP-A2- 1 938 847      WO-A1-00/02604
WO-A2-00/38761      GB-A- 2 276 566
US-A- 5 399 157**

**Beschreibung**

Die Erfindung betrifft eine Vorrichtung und ein Verfahren zur Erkennung der Richtung der Flüssigkeitsströmung durch einen Dialysator, der eine erste Kammer, die einen ersten und zweiten Anschluss hat, und eine zweite Kammer aufweist, die einen ersten und zweiten Anschluss hat, wobei die erste Kammer und die zweite Kammer von einer semipermeablen Membran voneinander getrennt sind. Darüber hinaus betrifft die Erfindung eine extrakorporale Blutbehandlungsvorrichtung mit einem extrakorporalen Blutkreislauf, der die Blutkammer eines durch eine semipermeable Membran in die Blutkammer und eine Dialysierflüssigkeitskammer unterteilten Dialysators einschließt, und mit einem Flüssigkeitssystem, das die Dialysierflüssigkeitskammer des Dialysators einschließt, wobei die extrakorporale Blutbehandlungsvorrichtung eine Vorrichtung zur Erkennung der Richtung der Flüssigkeitsströmung durch den Dialysator aufweist.

Es sind verschiedene Arten von Blutbehandlungsvorrichtungen bekannt. Zu den bekannten Blutbehandlungsvorrichtungen zählen beispielsweise die Vorrichtungen zur Hämodialyse, Hämofiltration und Hämodiafiltration. Während der Blutbehandlung strömt das Blut des Patienten in einem extrakorporalen Blutkreislauf durch eine Blutbehandlungseinheit. Bei den Vorrichtungen zur Hämodialyse, Hämofiltration und Hämodiafiltration ist die Blutbehandlungseinheit ein Dialysator oder Filter, der durch eine semipermeable Membran in eine Blutkammer und eine Dialysierflüssigkeitskammer getrennt ist. Während der Blutbehandlung strömt das Blut durch die Blutkammer, während die Dialysierflüssigkeit durch die Dialysierflüssigkeitskammer strömt. Eine effektive Blutbehandlung setzt voraus, dass Blut und Dialysierflüssigkeit entlang der Membran des Dialysators oder Filters in entgegengesetzten Richtungen strömen. Bei gleicher Strömungsrichtung ist die Blutbehandlung weniger effektiv. Daher wird in der Praxis der Dialysator oder Filter nicht im Gleichstrom, sondern Gegenstrom betrieben.

Der Dialysator oder Filter ist eine austauschbare Einheit, die mit dem Flüssigkeitssystem der Blutbehandlungsvorrichtung verbunden ist. Das Flüssigkeitssystem der bekannten

Blutbehandlungsvorrichtungen umfasst ein Leitungssystem mit einem ersten und einem zweiten Leitungsabschnitt für den Anschluss des Dialysators. Zum Anschluss des Dialysators an das Flüssigkeitssystem wird der erste Leitungsabschnitt mit dem Einlass der Dialysierflüssigkeitskammer und der zweite Leitungsabschnitt mit dem Auslass der Dialysierflüssigkeitskammer des Dialysators verbunden. Der Anschluss des Dialysators erfolgt mit bekannten Anschlussstücken, zu denen die bekannten Hansenkupplungen zählen.

Die Hersteller von Dialysatoren und Blutbehandlungsvorrichtungen sehen eine farbliche Kodierung des Ein- und Auslasses des Dialysators sowie der mit dem Ein- und Auslass zu verbindenden Hansenkupplungen vor, um den Anwender den Anschluss im Gegenstrom-Betrieb zu erleichtern. Diese farbliche Kodierung ist allerdings nicht bei allen Herstellern einheitlich. Daher besteht die Gefahr, dass die Anschlüsse verwechselt werden. Dies wird nachfolgend als nicht ordnungsgemäßer Anschluss bezeichnet.

Wenn der Dialysator nicht im Gegenstrom, sondern Gleichstrom betrieben wird, kann die Effektivität der Blutbehandlung für den Patienten nicht ausreichend sein. Dies ist insbesondere dann problematisch, wenn ein nicht ordnungsgemäßer Anschluss des Dialysators unbemerkt bleiben sollte. Dann besteht die Gefahr, dass der Patient auf Dauer mit nicht ausreichender Effektivität behandelt wird.

Aus der GB 2 276 566 A1 ist eine Vorrichtung zur extrakorporalen Blutbehandlung bekannt, die über eine Einrichtung zum Umkehren der Strömungsrichtung des in die Blutkammer des Dialysators fließenden Bluts verfügt. Die WO 00/02604 A1 beschreibt eine Dialysevorrichtung, die eine Einrichtung zur Bestimmung der Dialysanz auf der Grundlage einer Veränderung der Konzentration eines bestimmten Stoffes in der Dialysierflüssigkeit aufweist.

Der Erfindung liegt die Aufgabe zugrunde, eine Vorrichtung und ein Verfahren zur Erkennung der Richtung der Flüssigkeitsströmung durch einen Dialysator anzugeben, um überprüfen zu können, ob der Dialysator im Gleichstrom oder Gegenstrom betrieben wird. Darüber hinaus ist eine Aufgabe der Erfindung, eine extrakorporale Blutbehandlungsvorrichtung zu schaffen, mit der die Sicherheit bei der Dialyse erhöht ist.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung und das erfindungsgemäße Verfahren erlauben die Überwachung des Anschlusses eines Dialysators an das Flüssigkeitssystem einer extrakorporalen Blutbehandlungsvorrichtung. Die Erkennung der Richtung der Flüssigkeitsströmung durch den Dialysator der extrakorporalen Blutbehandlungsvorrichtung beruht auf der Veränderung einer physikalischen und/oder chemischen Eigenschaft einer in die eine Kammer des Dialysators strömenden Flüssigkeit und der Messung der Veränderung der physikalischen und/oder chemischen Eigenschaft der aus der einen Kammer des Dialysators strömenden Flüssigkeit. Die auf die Veränderung der physikalischen und/oder chemischen Eigenschaft stromauf der einen Kammer des Dialysators zurückzuführende Veränderung der physikalischen und/oder chemischen Eigenschaft der Flüssigkeit stromab der einen Kammer des Dialysators wird vor und nach der Umkehrung der Strömungsrichtung der Flüssigkeit durch die eine Kammer des Dialysators gemessen. Dabei wird die andere Kammer des Dialysators von einer zweiten Flüssigkeit durchströmt, deren Strömungsrichtung unverändert bleibt.

Die Flussumkehr kann im gesamten Flüssigkeitssystem oder nur in Teilen des Flüssigkeitssystems erfolgen. Zur Flussumkehr im gesamten Flüssigkeitssystem kann die Förderrichtung der Förderpumpe im Flüssigkeitssystem umgekehrt werden. Zur Flussumkehr nur in Teilen des Flüssigkeitssystems kann eine nach Art einer Weiche ausgebildete Ventilanordnung Verwendung finden.

Wenn die Veränderung der physikalischen und/oder chemischen Eigenschaft der ersten Flüssigkeit stromauf der einen Kammer des Dialysators bekannt ist, braucht die Veränderung der physikalischen und/oder chemischen Eigenschaft der ersten Flüssigkeit nicht gemessen zu werden. Für den Fall, dass die physikalische und/oder chemische Eigenschaft der ersten Flüssigkeit stromauf der einen Kammer des Dialysators jedoch nicht bekannt sein sollte, wird die physikalische und/oder chemische Eigenschaft der ersten Flüssigkeit sowohl stromauf als auch stromab des Dialysators gemessen.

Die Veränderung der physikalischen und/oder chemischen Eigenschaft kann durch eine Erhöhung und/oder Verringerung der physikalischen und/oder chemischen Eigenschaft erfolgen, wobei die Veränderung der physikalischen und/oder chemischen Eigenschaft vor und nach der Umkehr der Strömungsrichtung um den gleichen Betrag oder unterschiedliche Beträge erfolgen kann. Vorzugsweise wird die physikalische und/oder chemische Eigenschaft der Flüssigkeit um den gleichen Betrag verändert, der vorzugsweise bekannt ist. In diesem Fall braucht die Veränderung der physikalischen und/oder chemischen Eigenschaft nur stromab der einen Kammer des Dialysators gemessen zu werden.

Die durch die eine Kammer des Dialysators strömende erste Flüssigkeit kann Dialysierflüssigkeit sein, während die durch die zweite Kammer des Dialysators strömende zweite Flüssigkeit Blut sein kann. In diesem Fall wird die Strömungsrichtung der Dialysierflüssigkeit in der einen Kammer des Dialysators umgekehrt. Es ist aber grundsätzlich auch möglich, die Strömungsrichtung des Bluts in der anderen Kammer des Dialysators umzukehren.

Wenn die eine Kammer des Dialysators von Dialysierflüssigkeit und die andere Kammer des Dialysators von Blut durchströmt werden, kann die Erkennung der Durchströmungsrichtung des Dialysators während der Blutbehandlung erfolgen. Es ist aber auch möglich, die Durchströmungsrichtung des Dialysators vor der Blutbehandlung zu überprüfen, wenn die Kammern des Dialysators von anderen Flüssigkeiten durchströmt werden. Eine der beiden Kammern des Dialysators kann beispielsweise von einer Spülflüssigkeit durchströmt werden.

Für die Erkennung der Durchströmungsrichtung des Dialysators ist grundsätzlich unerheblich, welche physikalische und/oder chemische Eigenschaft verändert wird. Vorzugsweise wird die Konzentration eines Stoffes, beispielsweise die Na-Konzentration, in einer der beiden Flüssigkeiten verändert. Es ist aber auch möglich, die Temperatur der Flüssigkeit zu verändern.

Die Veränderung der Stoffmenge kann leicht in der Dialysataufbereitung der extrakorporalen Blutbehandlungsvorrichtung erfolgen. Auch die Temperatur der Dialysierflüssigkeit kann in der Dialysataufbereitung verändert werden. Die Messung der Konzentration eines Stoffes oder der Temperatur der Flüssigkeit kann mit den bekannten Sensoren erfolgen, die in den bekannten Blutbehandlungsvorrichtungen im Allgemeinen ohnehin vorhanden sind. Daher kann das erfindungsgemäße Verfahren bzw. die erfindungsgemäße Vorrichtung leicht in die bekannten Blutbehandlungsvorrichtungen implementiert werden.

Eine bevorzugte Ausführungsform der Erfindung sieht vor, das Integral über die physikalische und/oder chemische Eigenschaft der in die erste Kammer des Dialysators strömenden Flüssigkeit und das Integral der aus der ersten Kammer des Dialysators strömenden Flüssigkeit vor und nach der Umkehr der Strömungsrichtung zu berechnen, wobei der Betrieb des Dialysators vor der Umkehr der Strömungsrichtung im Gleichstrom oder im Gegenstrom auf der Grundlage eines Vergleichs der Differenz von den beiden Integralwerten nach der Umkehr der Strömungsrichtung mit der Differenz von den beiden Integralwerten vor der Umkehr der Strömungsrichtung bestimmt wird. Vorzugsweise wird der Quotient von der Differenz von den beiden Integralwerten nach der Umkehr der Strömungsrichtung und der Differenz von den beiden Integralwerten vor der Umkehr der Strömungsrichtung berechnet, wobei auf einen Betrieb des Dialysators vor der Umkehr der Strömungsrichtung im Gleichstrom geschlossen wird, wenn der Quotient größer als 1 ist oder auf einen Betrieb des Dialysators vor der Umkehr der Strömungsrichtung im Gegenstrom geschlossen wird, wenn der Quotient kleiner als 1 ist. Zur Berechnung des Integrals über die physikalische und/oder chemische Eigenschaft wird die Fläche berechnet, die von dem Grafen, der die physikalische und/oder chemische Eigenschaft in Abhängigkeit von der Zeit beschreibt, und der Basislinie eingeschossen wird, die den konstanten Betrag der physikalischen und/oder chemischen Eigenschaft darstellt. Die Schnittpunkte dieses Grafen mit der parallel zur Zeitachse verlaufenden Basislinie definieren die Integrationsgrenzen. Es wird also nur der Puls integriert, der auf die Basislinie aufgesetzt ist.

Wenn ein ordnungsgemäßer Betrieb des Dialysators der Betrieb im Gegenstrom ist, wird vorzugsweise ein Alarm, vorzugsweise ein optischer und/oder akustischer und/oder taktiler Alarm gegeben, wenn festgestellt wird, dass vor der Umkehr der Strömungsrichtung auf einen Betrieb des Dialysators im Gleichstrom geschlossen wird. Bei einem nicht ordnungsgemäßen Betrieb wird vorzugsweise ein Steuersignal für einen Eingriff in die Maschinensteuerung erzeugt. Der Eingriff in die Maschinensteuerung kann darin liegen, dass die Durchführung der Blutbehandlung verhindert wird. Dadurch ist sichergestellt, dass die Blutbehandlung nur bei ordnungsgemäßem Anschluss des Dialysators möglich ist. Es ist aber auch möglich, als Eingriff in die Maschinensteuerung die Flussrichtung umzukehren, so dass der Dialysator dann ordnungsgemäß betrieben wird.

Im Folgenden wird ein Ausführungsbeispiel der Erfindung unter Bezugnahme auf die Zeichnungen näher erläutert.

Es zeigen:

Fig. 1 in stark vereinfachter schematischer Darstellung die wesentlichen Komponenten einer extrakorporalen Blutbehandlungsvorrichtung,

Fig. 2 die Leitfähigkeit der Dialysierflüssigkeit stromauf und stromab der einen Kammer des im Gegenstrom betriebenen Dialysators als Funktion der Zeit und

Fig. 3 die Leitfähigkeit der Dialysierflüssigkeit stromauf und stromab der einen Kammer des im Gleichstrom betriebenen Dialysators.

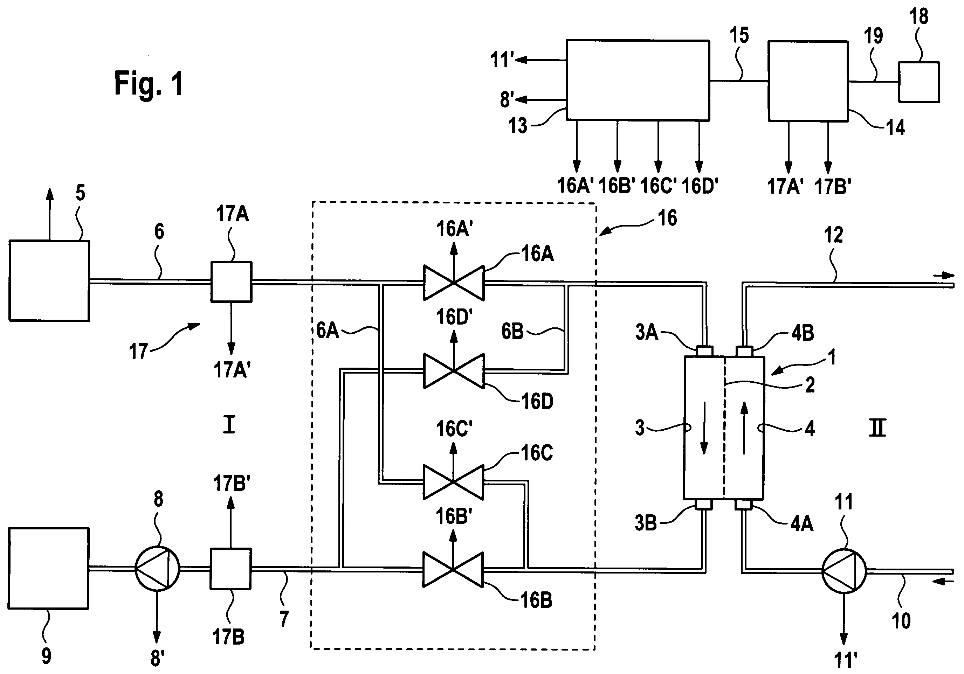

Fig. 1 zeigt in stark vereinfachter schematischer Darstellung nur die für die Erfindung wesentlichen Komponenten einer extrakorporalen Blutbehandlungsvorrichtung. Bei dem vorliegenden Ausführungsbeispiel ist die Vorrichtung zur Erkennung der Richtung der Flüssigkeitsströmung durch den Dialysator der extrakorporalen Blutbehandlungsvorrichtung Bestandteil der Blutbehandlungsvorrichtung. Die Vorrichtung zur Erkennung der Durchströmungsrichtung des Dialysators kann aber auch eine separate Einheit bilden.

Die extrakorporale Blutbehandlungsvorrichtung, die in dem vorliegenden Ausführungsbeispiel eine Hämodialysevorrichtung ist, weist einen Dialysator 1 auf, der durch eine semipermeable Membran 2 in eine erste Kammer 3 und eine zweite Kammer 4 getrennt ist. Die erste Kammer 3 weist einen ersten Anschluss 3A und einen zweiten Anschluss 3B auf, während die zweite Kammer einen ersten Anschluss 4A und einen zweiten Anschluss 4B aufweist. Die erste Kammer ist bei dem vorliegenden Ausführungsbeispiel die Dialysierflüssigkeitskammer 3, während die zweite Kammer die Blutkammer 4 ist.

Das Flüssigkeitssystem verfügt über eine nur schematisch dargestellte Einrichtung 5, mit der aus Wasser und Konzentraten frische Dialysierflüssigkeit hergestellt wird. Die Einrichtung 5 zur Bereitstellung frischer Dialysierflüssigkeit erlaubt eine kurzfristige Veränderung, insbesondere Erhöhung der Konzentratzusammensetzung, um einen Konzentratbolus zu erzeugen. Darüber hinaus erlaubt die Einrichtung 5 die kurzfristige Veränderung, insbesondere Erhöhung der Temperatur der Dialysierflüssigkeit, um einen Temperaturbolus zu erzeugen.

Die Einrichtung 5 zur Bereitstellung frischer Dialysierflüssigkeit ist über eine erste Dialysierflüssigkeitsleitung 6 mit dem ersten Anschluss 3A der Dialysierlfüssigkeitskammer 3 verbunden. Von dem zweiten Anschluss 3B der Dialysierflüssigkeitskammer 3 führt eine zweite Dialysierflüssigkeitsleitung 7, in die eine Dialysierflüssigkeitspumpe 8 geschaltet ist, zu einem Ablauf 9. Dieser Teil der Blutbehandlungsvorrichtung stellt das Dialysierflüssigkeitssystem I dar.

Von dem Patienten führt eine arterielle Blutleitung 10, in die eine Blutpumpe 11 geschaltet ist, zu dem ersten Anschluss 4A der Blutkammer 4, während von dem zweiten Anschluss 4B der Blutkammer 4 eine venöse Blutleitung 12 abgeht, die zurück zum Patienten führt. Dieser Teil der Blutbehandlungsvorrichtung stellt den extrakorporalen Blutkreislauf II dar.

Während der extrakorporalen Blutbehandlung wird die Dialysierflüssigkeitskammer 3 von Dialysierflüssigkeit und die Blutkammer 4 von Blut durchströmt. Dialysierflüssigkeit und Blut strömen dabei entlang der Membran 2 des Dialysators. Um die Effizienz der Behandlung zu steigern, wird der Dialysator 1 im Allgemeinen im Gegenstrom betrieben. Dabei strömen Dialysierflüssigkeit und Blut entlang der Membran in entgegengesetzter Richtung. Der Dialysator kann aber grundsätzlich auch im Gleichstrom betrieben werden.

Die Blutbehandlungsvorrichtung verfügt über eine zentrale Steuereinheit 13, die über Steuerleitungen 8', 11' mit der Dialysierflüssigkeitspumpe 8 und der Blutpumpe 11 verbunden ist.

Die erste und zweite Dialysierflüssigkeitsleitung 6, 7 sind Schlauchleitungen, an denen der Dialysator 1 angeschlossen wird. Zum Anschluss der Schlauchleitungen 6, 7 an den Anschlüssen 3A, 3B des Dialysators 1 dienen nicht dargestellte Anschlussstücke, insbesondere Hansenkupplungen, die im Allgemeinen farblich kodiert sind.

Die Vorrichtung zum Erkennen der Durchströmungsrichtung des Dialysators 1, die bei dem vorliegenden Ausführungsbeispiel Bestandteil der Blutbehandlungsvorrichtung ist, verfügt über eine Rechen- und Auswerteinheit 14, die über eine Datenleitung 15 mit der zentralen Steuereinheit 13 der Blutbehandlungsvorrichtung verbunden ist. Die Rechen- und Auswerteinheit 14 kann aber auch Bestandteil der Steuereinheit 13 sein.

Die Erkennung der Durchströmungsrichtung des Dialysators 1 setzt die Umkehr der Flussrichtung durch den Dialysator voraus. Hierzu sind Mittel 16 zum Umkehren der Flussrichtung vorgesehen, die eine Anordnung von Ventilen 16A, 16B, 16C, 16D umfassen. Die Ventile sind vorzugsweise elektromagnetisch oder pneumatisch betätigbare Ventile, die über Steuerleitungen 16A', 16B', 16C', 16D' von der zentralen Steuereinheit 13 der Blutbehandlungsvorrichtung angesteuert werden.

Das Ventil 16A ist in der ersten Dialysierflüssigkeitsleitung 6 und das zweite Ventil 16B in der zweiten Dialysierflüssigkeitsleitung 7 angeordnet. Stromauf des ersten Ventils 16A zweigt von der ersten Dialysierflüssigkeitsleitung 6 ein erster Leitungszweig 6A ab, der zu der zweiten Dialysierflüssigkeitsleitung 7 stromauf des zweiten Ventils 16B führt. In den ersten Leitungszweig 6A ist das dritte Ventil 16C geschaltet. Stromab des ersten Ventils 16A zweigt ein zweiter Leitungszweig 6B von der ersten Dialysierflüssigkeitsleitung 6 ab, der zu der zweiten Dialysierflüssigkeitsleitung 7 stromab des zweiten Ventils 16B führt. In den zweiten Leitungszweig 6B ist das vierte Ventil 16D geschaltet. Dabei beziehen sich die Begriffe stromauf und stromab der Ventile auf die Flussrichtung, wenn die Flüssigkeitsströmung nicht umgekehrt ist

Im Normal-Betrieb wird der Dialysator 1 im Gegenstrom betrieben. Hierzu öffnet die zentrale Steuereinheit 13 das erste und zweite Ventil 16A, 16B und schließt das dritte und vierte Ventil 16C, 16D. Folglich ist der erste Anschluss 3A der Einlass und der zweite Anschluss 3B der Auslass der Dialysierflüssigkeitskammer 3. Zur Umkehr der Strömungsrichtung schließt die Steuereinheit 13 das erste und zweite Ventil 16A, 16B und öffnet das dritte und vierte Ventil 16C, 16D. Dann ist der erste Anschluss 3A der Auslass und der zweite Anschluss 3B der Einlass der Dialysierflüssigkeitskammer 3.

Die Vorrichtung zum Erkennen der Durchströmungsrichtung des Dialysators verfügt weiterhin über Mittel zum Messen einer physikalischen und/oder chemischen Eigenschaft der Dialysierflüssigkeit. Bei dem vorliegenden Ausführungsbeispiel ist die physikalisch und/oder chemische Eigenschaft der Dialysierflüssigkeit entweder die Konzentration eines Stoffes in der Dialysierflüssigkeit, bspw. die Natrium-Konzentration, oder die Temperatur der Dialysierflüssigkeit. Zum Messen der physikalischen und/oder chemischen Eigenschaft sind Mittel 17 vorgesehen, die einen ersten Sensor 17A und einen zweiten Sensor 17B umfassen. Der erste Sensor 17A misst zur Bestimmung der Na-Konzentration die Leitfähigkeit der Dialysierflüssigkeit in der ersten Dialysierflüssigkeitsleitung 6 stromauf des Dialysators 1, während der zweite Sensor 17B die Leitfähigkeit der Dialysierflüssigkeit in der zweiten Dialysierflüssigkeitsleitung 7 stromab des Dialysators 1 misst, wenn die Flussrichtung nicht umgekehrt ist. Anstelle von Leitfähigkeitssensoren sind bei der alternativen Ausführungsform Temperatursensoren 17A, 17B vorgesehen. Die Sensoren 17A, 17B sind über Datenleitungen 17A', 17B' mit der Rechen- und Auswerteinheit 14 verbunden.

Darüber hinaus ist eine Alarmeinheit 18 vorgesehen, die über eine Datenleitung 19 mit der Rechen- und Auswerteinheit 14 verbunden ist. Die Alarmeinheit 18 gibt einen optischen und/oder akustischen und/oder taktilen Alarm, wenn ein nicht ordnungsgemäßer Betrieb des Dialysators 1 festgestellt wird.

Zunächst werden die theoretischen Grundlagen der Erkennung der Durchströmungsrichtung des Dialysators erläutert.

Durch kurzfristige Veränderung der Konzentratzusammensetzung oder der Temperatur der Dialysierflüssigkeit im Dialysierflüssigkeitskreislauf I wird ein Konzentratbolus bzw. Temperaturbolus erzeugt, der mit den Sensoren 17A und 17B gemessen wird. Die Menge eines Stoffes einer bestimmten Konzentration $c_{di}$, die stromauf des Dialysators gemessen wird, teilt sich auf in einen Anteil $c_{bo}$, der auf die Blutseite übertritt, und einen Anteil $c_{do}$, der stromab des Dialysators über eine Messzeit $\Delta t$ gemessen werden kann:

$$[1] \qquad Q_D \int\limits_{\Delta t} c_{di}\, dt = Q_D \int\limits_{\Delta t} c_{do}\, dt + Q_B \int\limits_{\Delta t} c_{bo}\, dt$$

Das Verhältnis der Integrale über die Konzentration stromab zu stromauf ist ein Maß für die Dialysance $\Psi$ der betreffenden Stoffmenge.

$$[2] \qquad \Rightarrow \qquad 1 - \frac{\int\limits_{\Delta t} c_{do}\, dt}{\int\limits_{\Delta t} c_{di}\, dt} = \frac{Q_B \int\limits_{\Delta t} c_{bo}\, dt}{Q_D \int\limits_{\Delta t} c_{di}\, dt} = \Psi$$

Die Flussrate, mit welcher die Stoffmenge auf der Dialysatseite transportiert wird, ist mit $Q_D$, und die Flussrate, mit welcher die Menge auf der Blutseite bewegt wird, ist mit $Q_B$ bezeichnet.

Die erhaltenen Stoffmengen nach Umkehr der Strömungsrichtung ohne Änderung von Dialysierflüssigkeits- und Blutfluss $Q_D$, $Q_B$ lauten:

$$[3] \qquad Q_D \int_{\Delta t} \hat{c}_{di} dt = Q_D \int_{\Delta t} \hat{c}_{do} dt + Q_B \int_{\Delta t} \hat{c}_{bo} dt$$

$$[4] \qquad => \quad 1 - \frac{\int_{\Delta t} \hat{c}_{do} dt}{\int_{\Delta t} \hat{c}_{di} dt} = \frac{Q_B \int_{\Delta t} \hat{c}_{bo} dt}{Q_D \int_{\Delta t} \hat{c}_{di} dt} = \hat{\Psi}$$

Das Verhältnis der Dialysance $\hat{\Psi}$ zu $\Psi$ liefert eine Zahl größer oder kleiner 1:

$$[5] \qquad \frac{\hat{\Psi}}{\Psi} = \frac{\left( \int_{\Delta t} \hat{c}_{di} dt - \int_{\Delta t} \hat{c}_{do} dt \right) \int_{\Delta t} c_{di} dt}{\left( \int_{\Delta t} c_{di} dt - \int_{\Delta t} c_{do} dt \right) \int_{\Delta t} \hat{c}_{di} dt}$$

Bei Änderung von Blut- bzw. Dialysierflüssigkeitsfluss sind die Gleichungen entsprechend anzupassen. Unter gleichen Rahmenbedingungen kann in der Regel $c_{di} = \hat{c}_{di}$ gesetzt werden, weil die Pulsform unter gleichen Rahmenbedingungen, beispielsweise bei gleichem Dialysatfluss und gleichen Schlauchlängen, gleich aussehen wird.

Ist das Verhältnis $\frac{\hat{\Psi}}{\Psi}$ kleiner 1, so ist die Strömung zwischen Dialysierflüssigkeits- und Blutfluss während der Messung von $\hat{\Psi}$ in der gleichen Richtung und während der Messung von $\Psi$ in entgegengesetzter Richtung. Folglich wird der Dialysator im Gegenstrom betrieben.

Ist das Verhältnis $\frac{\hat{\Psi}}{\Psi}$ größer 1, so ist die Strömung zwischen Dialysierflüssigkeits- und Blutfluss während der Messung von $\hat{\Psi}$ in entgegengesetzter Richtung und während der Messung von $\Psi$ in der gleichen Richtung. Folglich wird der Dialysator im Gleichstrom betrieben.

In Analogie zur Leitfähigkeitsmessung kann die Temperaturmessung erfolgen, um die Durchströmung des Dialysators zu analysieren. Hierzu wird lediglich anstelle der Leitfähigkeit die Temperatur verändert und gemessen.

Nachfolgend werden die einzelnen Schritte zur Durchführung der Messung im Einzelnen beschrieben.

Es wird davon ausgegangen, dass der Dialysator im Gegenstrom betrieben werden soll. Der Gegenstrom-Betrieb ist also der Normal-Betrieb. Dies soll im vorliegenden Ausführungsbeispiel überprüft werden. Für den Gegenstrom-Betrieb sind die Ventile 16A, 16B geöffnet und die Ventile 16C, 16D geschlossen.

Zur Überprüfung des Dialysator-Anschlusses wird zunächst die Flussrichtung umgekehrt. Hierzu schließt die zentrale Steuereinheit 13 die Ventile 16A, 16B und öffnet die Ventile 16C, 16D. Daraufhin wird ein Konzentrat-Bolus erzeugt. Der Konzentrat-Bolus kann durch Änderung der Fördermenge der nicht dargestellten Förderpumpe der Einrichtung 5 zur Bereitstellung von Dialysierflüssigkeit erzeugt werden. Die Rechen- und Auswerteinheit 14 erfasst mit dem Leitfähigkeitssensor 17A stromauf des Dialysators 1 und dem Leitfähigkeitssensor 17B stromab des Dialysators die Leitfähigkeit. Die Leitfähigkeit kann in einem vorgegebenen Zeitintervall, in dem der Leitfähigkeitsbolus erzeugt wird, kontinuierlich oder diskontinuierlich gemessen werden. Die Messwerte werden in einem nicht dargestellten Speicher der Rechen- und Auswerteinheit 14 abgelegt.

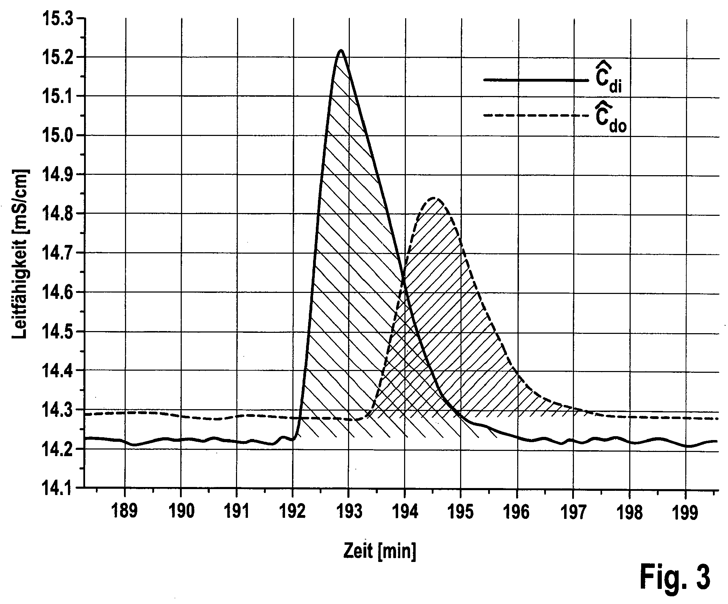

Fig. 3 zeigt den Leitfähigkeitsbolus, der stromauf und stromab der Dialysierflüssigkeitskammer 3 des Dialysators 1 bei umgekehrter Strömungsrichtung erfasst wird. In Fig. 3 ist mit $\hat{c}_{di}$ die Leitfähigkeit stromauf und mit $\hat{c}_{do}$ die Leitfähigkeit stromab des Dialysators bezeichnet. Es zeigt sich, dass dem Leitfähigkeitsbolus $\hat{c}_{di}$ stromauf des Dialysators zeitlich der Leitfähigkeitsbolus $\hat{c}_{do}$ stromab des Dialysators folgt.

Daraufhin wird die Flussrichtung der Dialysierflüssigkeit wieder umgekehrt. Hierzu werden die Ventile 16C, 16D geschlossen und die Ventile 16A, 16B geöffnet. Nach der Umkehr der Flussrichtung wird wieder ein Konzentratbolus erzeugt. Die Leitfähigkeit der Dialysierflüssigkeit wird stromauf des Dialysators mit dem Sensor 17A und stromab des Dialysators mit dem Sensor 17B gemessen. Die Leitfähigkeitswerte werden wieder in dem Speicher der Rechen- und Auswerteinheit 14 abgelegt.

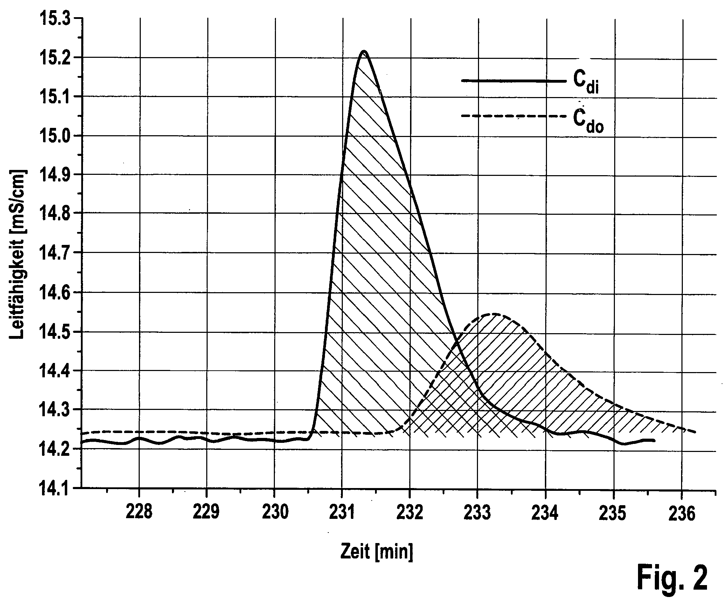

Fig. 2 zeigt den Leitfähigkeitsbolus $c_{di}$ stromauf und den Leitfähigkeitsbolus $c_{do}$ stromab des Dialysators nach der Umkehr der Strömungsrichtung, die dem Normal-Betrieb des Dialysators entspricht.

Nunmehr führt die Rechen- und Auswerteinheit 14 eine Integration der mit den Sensoren 17A, 17B gemessenen Leitfähigkeit über die Zeit durch. Die Rechen- und Auswerteinheit 14 berechnet das Integral der Leitfähigkeit stromauf des Dialysators und das Integral der Leitfähigkeit stromab des Dialysators bei der ersten Messung mit umgekehrter Strömungsrichtung und berechnet das Integral stromauf des Dialysators und das Integral stromab des Dialysators bei der zweiten Messung mit dem Dialysator im Normal-Betrieb. Das Integral über die Zeit wird in den Figuren durch eine schraffierte Fläche dargestellt, die von der jeweiligen Kurve und der jeweiligen Grundlinie begrenzt wird. Mit den berechneten Integralwerten stromauf und stromab des Dialysators nach der Umkehr der Strömungsrichtung (erste Messung) und vor der Umkehr der Strömungsrichtung (zweite Messung) wird der Quotient $\frac{\hat{\Psi}}{\Psi}$ nach Gleichung (5) berechnet.

Den Quotienten $\frac{\hat{\Psi}}{\Psi}$ vergleicht die Rechen- und Auswerteinheit 14 mit dem Wert 1. Wenn der Quotient $\frac{\hat{\Psi}}{\Psi}$ kleiner als 1 ist, stellt die Rechen- und Auswerteinheit 14 fest, dass der Dialysator im Gegenstrom betrieben worden ist. Für den Fall, dass das Verhältnis größer als 1 ist, stellt die Rechen- und Auswerteinheit 14 fest, dass der Dialysator im Gleichstrom betrieben worden ist.

Im vorliegenden Fall eines ordnungsgemäßen Anschlusses des Dialysators ist der Quotient $\frac{\hat{\Psi}}{\Psi}$ kleiner als 1.

Wenn Dialysator nicht ordnungsgemäß an die Dialysierflüssigkeitsleitungen 6, 7 angeschlossen sein sollte, d.h. die Anschlüsse vertauscht sein sollten, stellt die Rechen- und Auswerteinheit fest, dass der Dialysator im Gleichstrom betrieben worden ist. Dann erzeugt die Rechen- und Auswerteinheit 14 ein Steuersignal, das die Alarmeinheit 18 empfängt. Die Alarmeinheit 18 gibt jetzt einen Alarm. Darüber hinaus erzeugt die Rechen- und Auswerteinheit 14 ein Steuersignal, das die zentrale Steuereinheit 13 empfängt. Daraufhin nimmt die Steuereinheit 13 einen Eingriff in die Maschinensteuerung vor. Dieser Eingriff kann darin liegen, dass die Durchführung der Blutbehandlung unterbrochen wird. Alternativ ist es möglich, die Flussrichtung durch Betätigung der entsprechenden Ventile 16A - 16D umzukehren, so dass der Dialysator im Gegenstrom betrieben wird.

Für das vorliegende Ausführungsbeispiel können die folgenden Zahlenwerte angenommen werden:

$$\text{Sei } c_{di} = \hat{c}_{di}, \int_{\Delta t} c_{di}\,dt = 100, \int_{\Delta t} c_{do}\,dt = 40 \text{ und } \int_{\Delta t} \hat{c}_{do}\,dt = 70 \text{ folgt gemäß Gleichung [5]:}$$

$$\frac{\hat{\Psi}}{\Psi} = \frac{(100-70)}{(100-40)} = \frac{30}{60} = 0.5 < 1$$

Bei dem zweiten Ausführungsbeispiel wird anstelle der Stoffkonzentration die Temperatur verändert. Die Berechung und der Vergleich der Integralwerte erfolgt analog zu dem ersten Ausführungsbeispiel.

**Patentansprüche**

1.  Vorrichtung zur Erkennung der Richtung der Flüssigkeitsströmung durch einen Dialysator (1), der eine erste Kammer (3), die einen ersten und zweiten Anschluss (3A, 3B) hat, und eine zweite Kammer (4) aufweist, die einen ersten und zweiten Anschluss (4A, 4B) hat, wobei die erste Kammer und die zweite Kammer von einer semipermeablen Membran (2) voneinander getrennt sind, mit

Mitteln (16) zum Umkehren der Strömungsrichtung, die derart ausgebildet sind, dass der Dialysator

zwischen einem Gleichstrom-Betrieb, in dem eine erste Flüssigkeit in den ersten Anschluss der ersten Kammer strömt und die erste Flüssigkeit aus dem zweiten Anschluss der ersten Kammer strömt, während die zweite Flüssigkeit in den ersten Anschluss der zweiten Kammer und die zweite Flüssigkeit aus dem zweiten Anschluss der zweiten Kammer strömt, und einem

Gegenstrom-Betrieb, in dem die erste Flüssigkeit in den zweiten Anschluss der ersten Kammer strömt und die erste Flüssigkeit aus dem ersten Anschluss der ersten Kammer strömt, während die zweite Flüssigkeit in den ersten Anschluss der zweiten Kammer und die zweite Flüssigkeit aus dem zweiten Anschluss der zweiten Kammer strömt, oder

zwischen dem Gegenstrom-Betrieb und dem Gleichstrom-Betrieb umschaltbar ist,

Mitteln (5) zum Verändern einer physikalischen und/oder chemischen Eigenschaft der in den ersten bzw. zweiten Anschluss der ersten Kammer strömenden Flüssigkeit,

Mitteln (17; 17B) zum Messen der physikalischen und/oder chemischen Eigenschaft der aus dem zweiten bzw. ersten Anschluss der ersten Kammer strömenden Flüssigkeit, und

einer mit den Mitteln (17) zum Messen der physikalischen und/oder chemischen Eigenschaft zusammenwirkenden Rechen- und Auswerteinheit (14), die derart ausgebildet ist, dass auf der Grundlage der vor der Umkehr der Strömungsrichtung und nach der Umkehr der Strömungsrichtung gemessenen Änderung der physikalischen und/oder chemischen Eigenschaft der aus dem zweiten bzw. ersten Anschluss der ersten Kammer strömenden ersten Flüssigkeit, die auf die Änderung der physikalischen und/oder chemischen Eigenschaft der in den ersten bzw. zweiten Anschluss der ersten Kammer strömenden ersten Flüssigkeit zurückzuführen ist, darauf geschlossen wird, ob der Dialysator vor der Umkehr der Strömungsrichtung im Gleichstrom-Betrieb oder im Gegenstrom-Betrieb betrieben worden ist.

2. Vorrichtung nach Anspruch 1, **dadurch gekennzeichnet, dass** die Mittel (17; 17A) zum Messen der physikalischen und/oder chemischen Eigenschaft der in den ersten bzw. zweiten Anschluss der ersten Kammer strömenden Flüssigkeit vorgesehen sind.

3. Vorrichtung nach Anspruch 2, **dadurch gekennzeichnet, dass** die Rechen- und Auswerteinheit (14) derart ausgebildet ist, dass

das Integral über die physikalische und/oder chemische Eigenschaft der in den ersten Anschluss der ersten Kammer strömenden ersten Flüssigkeit und das Integral der aus dem zweiten Anschluss der ersten Kammer strömenden ersten Flüssigkeit vor der Umkehr der Flussrichtung berechnet werden und

das Integral über die physikalische und/oder chemische Eigenschaft der in den zweiten Anschluss der ersten Kammer strömenden ersten Flüssigkeit und das Integral der aus dem ersten Anschluss der ersten Kammer strömenden ersten Flüssigkeit nach der Umkehr der Flussrichtung berechnet werden, wobei

der Betrieb des Dialysators vor der Umkehr der Strömungsrichtung im Gleichstrom oder im Gegenstrom auf der Grundlage eines Vergleichs der Differenz von den beiden Integralwerten nach der Umkehr der Strömungsrichtung mit der Differenz von den beiden Integralwerten vor der Umkehr der Strömungsrichtung bestimmt wird.

4. Vorrichtung nach Anspruch 3, **dadurch gekennzeichnet, dass** die Rechen- und Auswerteinheit (14) derart ausgebildet ist, dass

der Quotient von der Differenz von den beiden Integralwerten nach der Umkehr der Strömungsrichtung und der Differenz von den beiden Integralwerten vor der Umkehr der Strömungsrichtung berechnet wird, wobei

auf einen Betrieb des Dialysators vor der Umkehr der Strömungsrichtung im Gleichstrom geschlossen wird, wenn der Quotient größer als 1 ist oder

auf einen Betrieb des Dialysators vor der Umkehr der Strömungsrichtung im Gegenstrom geschlossen wird, wenn der Quotient kleiner als 1 ist.

5. Vorrichtung nach einem der Ansprüche 1 bis 4, **dadurch gekennzeichnet, dass** die erste Flüssigkeit Dialysierflüssigkeit ist.

6. Vorrichtung nach einem der Ansprüche 1 bis 5, **dadurch gekennzeichnet, dass** die zweite Flüssigkeit Blut ist.

7. Vorrichtung nach einem der Ansprüche 1 bis 6, **dadurch gekennzeichnet, dass** die physikalische und/oder chemische Eigenschaft die Konzentration eines Stoffes in der Flüssigkeit ist, wobei die Mittel (17) zum Messen der physikalischen und/oder chemischen Eigenschaft Mittel zum Messen der Stoffkonzentration sind.

8. Vorrichtung nach einem der Ansprüche 1 bis 6, **dadurch gekennzeichnet, dass** die physikalische und/oder chemische Eigenschaft die Temperatur der Flüssigkeit ist, wobei die Mittel (17) zum Messen der physikalischen und/oder chemischen Eigenschaft Mittel zum Messen der Temperatur sind.

9. Vorrichtung nach einem der Ansprüche 1 bis 8, **dadurch gekennzeichnet, dass** die Rechen- und Auswerteinheit (14) mit einer Alarmeinheit (18) zusammenwirkt, die einen optischen und/oder akustischen und/oder taktilen Alarm gibt, wenn die Rechen- und Auswerteinheit vor der Umkehr der Strömungsrichtung einen Betrieb des Dialysators im Gleichstrom festgestellt hat.

10. Vorrichtung nach einem der Ansprüche 1 bis 9, **dadurch gekennzeichnet, dass** die Rechen- und Auswerteinheit (14) ein Steuersignal für einen Eingriff in die Maschinensteuerung erzeugt, wenn die Rechen- und Auswerteinheit vor der Umkehr der Strömungsrichtung einen Betrieb des Dialysators im Gleichstrom festgestellt hat.

11. Vorrichtung nach Anspruch 10, **dadurch gekennzeichnet, dass** das Steuersignal für den Eingriff in die Maschinensteuerung ein die Mittel (16) zum Umkehren der Flussrichtung aktivierendes Steuersignal ist.

12. Extrakorporale Blutbehandlungsvorrichtung mit einem extrakorporalen Blutkreislauf (II), der die Blutkammer (4) eines durch eine semipermeable Membran (2) in die Blutkammer und eine Dialysierflüssigkeitskammer (3) unterteilten Dialysator (1) einschließt, und einem Dialysierflüssigkeitssystem (I), das die Dialysierflüssigkeitskammer einschließt, **dadurch gekennzeichnet, dass** die extrakorporale Blutbehandlungsvorrichtung eine Vorrichtung zur Erkennung der Richtung der Flüssigkeitsströmung durch einen Dialysator nach einem der Ansprüche 1 bis 11 aufweist.

13. Verfahren zur Erkennung der Richtung der Flüssigkeitsströmung durch einen Dialysator, der eine erste Kammer, die einen ersten und zweiten Anschluss hat, und eine zweite Kammer aufweist, die einen ersten und zweiten Anschluss hat, wobei die erste Kammer und die zweite Kammer von einer semipermeablen Membran voneinander getrennt sind, wobei
eine physikalische und/oder chemische Eigenschaft einer in den ersten Anschluss der ersten Kammer strömenden ersten Flüssigkeit verändert wird, während eine zweite Flüssigkeit in den ersten Anschluss der zweiten Kammer des Dialysators und aus dem zweiten Anschluss der zweiten Kammer des Dialysators strömt,
die physikalische und/oder chemische Eigenschaft der aus dem zweiten Anschluss der ersten Kammer strömenden ersten Flüssigkeit, die auf die Änderung der physikalischen und/oder chemischen Eigenschaft der in den ersten Anschluss der ersten Kammer strömenden ersten Flüssigkeit zurückzuführen ist, gemessen wird,
dass die Strömungsrichtung derart umgekehrt wird, dass die erste Flüssigkeit in den zweiten Anschluss der ersten Kammer strömt und die erste Flüssigkeit aus dem ersten Anschluss der ersten Kammer strömt, und
auf der Grundlage der vor der Umkehr der Strömungsrichtung und nach der Umkehr der Strömungsrichtung gemessenen Änderung der physikalischen und/oder chemischen Eigenschaft der aus dem zweiten bzw. ersten Anschluss der ersten Kammer strömenden ersten Flüssigkeit, die auf die Änderung der physikalischen und/oder chemischen Eigenschaft der in den ersten bzw. zweiten Anschluss der ersten Kammer strömenden ersten Flüssigkeit zurückzuführen ist, darauf geschlossen wird, ob der Dialysator vor der Umkehr der Strömungsrichtung im Gleichstrom-Betrieb oder im Gegenstrom-Betrieb betrieben worden ist.

14. Verfahren nach Anspruch 13, **dadurch gekennzeichnet, dass** die physikalische und/oder chemische Eigenschaft der in den ersten bzw. zweiten Anschluss der ersten Kammer strömenden Flüssigkeit gemessen wird.

15. Verfahren nach Anspruch 14, **dadurch gekennzeichnet, dass** das Integral über die physikalische und/oder chemische Eigenschaft der in den ersten Anschluss der ersten Kammer strömenden ersten Flüssigkeit und das Integral der aus dem zweiten Anschluss der ersten Kammer strömenden ersten Flüssigkeit vor der Umkehr der Flussrichtung berechnet werden und
das Integral über die physikalische und/oder chemische Eigenschaft der in den zweiten Anschluss der ersten Kammer strömenden ersten Flüssigkeit und das Integral der aus dem ersten Anschluss der ersten Kammer strömenden ersten Flüssigkeit nach der Umkehr der Flussrichtung berechnet werden, wobei
der Betrieb des Dialysators vor der Umkehr der Strömungsrichtung im Gleichstrom oder im Gegenstrom auf der Grundlage eines Vergleichs der Differenz von den beiden Integralwerten nach der Umkehr der Strömungsrichtung mit der Differenz von den beiden Integralwerten vor der Umkehr der Strömungsrichtung bestimmt wird.

16. Verfahren nach Anspruch 15, **dadurch gekennzeichnet, dass** der Quotient von der Differenz von den beiden Integralwerten nach der Umkehr der Strömungsrichtung und der Differenz von den beiden Integralwerten vor der Umkehr der Strömungsrichtung berechnet wird, wobei
auf einen Betrieb des Dialysators vor der Umkehr der Strömungsrichtung im Gleichstrom geschlossen wird, wenn der Quotient größer als 1 ist oder
auf einen Betrieb des Dialysators vor der Umkehr der Strömungsrichtung im Gegenstrom geschlossen wird, wenn der Quotient kleiner als 1 ist.

17. Verfahren nach einem der Ansprüche 13 bis 16, **dadurch gekennzeichnet, dass** die erste Flüssigkeit Dialysierflüssigkeit ist.

18. Verfahren nach einem der Ansprüche 13 bis 17, **dadurch gekennzeichnet, dass** die zweite Flüssigkeit Blut ist.

19. Verfahren nach einem der Ansprüche 13 bis 18, **dadurch gekennzeichnet, dass** die physikalische und/oder chemische Eigenschaft die Konzentration eines Stoffes in der Flüssigkeit ist.

20. Verfahren nach einem der Ansprüche 13 bis 18, **dadurch gekennzeichnet, dass** die physikalische und/oder chemische Eigenschaft die Temperatur der Flüssigkeit ist.

21. Verfahren nach einem der Ansprüche 13 bis 19, **dadurch gekennzeichnet, dass** ein optischer und/oder akustischer und/oder taktiler Alarm gegeben wird, wenn vor der Umkehr der Strömungsrichtung ein Betrieb des Dialysators im Gleichstrom festgestellt wird.

**Claims**

1. A device for detecting the direction of the fluid flow through a dialyser (1), which comprises a first chamber (3) which has a first and second connection (3A, 3B) and a second chamber (4) which has a first and second connection (4A, 4B), wherein the first chamber and the second chamber are separated from one another by a semipermeable membrane (2),
with
means (16) for reversing the flow direction which are constituted such that
the dialyser
can be switched over between an equi-directional flow operation, wherein a first fluid flows into the first connection of the first chamber and the first fluid flows out of the second connection of the first chamber, whilst the second fluid flows into the first connection of the second chamber and the second fluid flows out of the second connection of the second chamber, and a
counter-flow operation, wherein the first fluid flows into the second connection of the first chamber and the first fluid flows out of the first connection of the first chamber, whilst the second fluid flows into the first connection of the second chamber and the second fluid flows out of the second connection of the second chamber,
or
between the counter-flow operation and the equi-directional flow operation,
means (5) for changing a physical and/or chemical property of the fluid flowing into the first and/or second connection of the first chamber,
means (17; 17B) for measuring the physical and/or chemical property of the fluid flowing out of the second and/or first connection of the first chamber, and
a computing and evaluation unit (14) cooperating with the means (17) for measuring the physical and/or chemical property, said computing and evaluation unit being constituted such that, on the basis of the change in the physical and/or chemical property of the first fluid flowing out of the second and/or first connection of the first chamber, said change being measured before the reversal of the flow direction and after the reversal of the flow direction and being able to be traced back to the change in the physical and/or chemical property of the first fluid flowing into the first and/or second connection of the first chamber, it is concluded whether the dialyser has been operated with an equi-directional flow operation or a counter-flow operation before the reversal of the flow direction.

2. The device according to claim 1, **characterised in that** the means (17; 17A) are provided for measuring the physical and/or chemical property of the fluid flowing into the first and/or second connection of the first chamber.

3. The device according to claim 2, **characterised in that** the computing and evaluation unit (14) is constituted such that the integral over the physical and/or chemical property of the first fluid flowing into the first connection of the first chamber and the integral of the first fluid flowing out of the second connection of the first chamber are calculated before the reversal of the flow direction and the integral over the physical and/or chemical property of the first fluid flowing into the second connection of the first chamber and the integral of the first fluid flowing out of the first connection of the first chamber are calculated after the reversal of the flow direction, wherein

the operation of the dialyser before the reversal of the flow direction with an equi-directional flow or with a counter-flow is determined on the basis of a comparison of the difference between the two integral values after the reversal of the flow direction with the difference between the two integral values before the reversal of the flow direction.

4. The device according to claim 3, **characterised in that** the computing and evaluation unit (14) is constituted such that the quotient of the difference between the two integral values after the reversal of the flow direction and the difference between the two integral values before the reversal of the flow direction is calculated, wherein

it is concluded that an operation of the dialyser before the reversal of the flow direction is with an equi-directional flow if the quotient is greater than 1 or

it is concluded that there is an operation of the dialyser before the reversal of the flow direction with a counter-flow if the quotient is less than 1.

5. The device according to any one of claims 1 to 4, **characterised in that** the first fluid is dialysing fluid.

6. The device according to any one of claims 1 to 5, **characterised in that** the second fluid is blood.

7. The device according to any one of claims 1 to 6, **characterised in that** the physical and/or chemical property is the concentration of a substance in the fluid, the means (17) for measuring the physical and/or chemical property being means for measuring the substance concentration.

8. The device according to any one of claims 1 to 6, **characterised in that** the physical and/or chemical property is the temperature of the fluid, the means (17) for measuring the physical and/or chemical property being means for measuring the temperature.

9. The device according to any one of claims 1 to 8, **characterised in that** the computing and evaluation unit (14) cooperates with an alarm unit (18), which emits an optical and/or acoustic and/or tactile alarm when the computing and evaluation unit has ascertained an operation of the dialyser with an equi-directional flow before the reversal of the flow direction.

10. The device according to any one of claims 1 to 9, **characterised in that** the computing and evaluation unit (14) generates a control signal for an intervention into the machine control when the computing and evaluation unit has ascertained an operation of the dialyser with an equi-directional flow before the reversal of the flow direction.

11. The device according to claim 10, **characterised in that** the control signal for the intervention into the machine control is a control signal activating the means (16) for reversing the flow direction.

12. An extracorporeal blood treatment apparatus with an extracorporeal blood circuit (II), which includes the blood chamber (4) of a dialyser (1) divided by a semipermeable membrane (2) into the blood chamber and a dialysing fluid chamber (3), and a dialysing fluid system (I), which includes the dialysing fluid chamber, **characterised in that** the extracorporeal blood treatment apparatus comprises a device for detecting the direction of the fluid flow through a dialyser according to any one of claims 1 to 11.

13. A method for detecting the direction of the fluid flow through a dialyser, which comprises a first chamber which has a first and second connection and a second chamber which has a first and second connection, wherein the first chamber and the second chamber are separated from one another by a semipermeable membrane, **characterised in that**

a physical and/or chemical property of a first fluid flowing into the first connection of the first chamber is changed, whilst a second fluid flows into the first connection of the second chamber of the dialyser and out of the second connection of the second chamber of the dialyser,

the physical and/or chemical property of the first fluid flowing out of the second connection of the first chamber, which physical and/or chemical property can be traced back to the change in the physical and/or chemical property of the first fluid flowing into the first connection of the first chamber, is measured,

that the flow direction is reversed such that the first fluid flows into the second connection of the first chamber and the first fluid flows out of the first connection of the first chamber and,

on the basis of the change in the physical and/or chemical property of the first fluid flowing out of the second and/or first connection of the first chamber, said change being measured before the reversal of the flow direction and after the reversal of the flow direction and being able to be traced back to the change in the physical and/or chemical property of the first fluid flowing into the first and/or second connection of the first chamber, it is concluded whether the dialyser has been operated in an equi-directional flow operation or in a counter-flow operation before the reversal of the flow direction.

14. The method according to claim 13, **characterised in that** the physical and/or chemical property of the fluid flowing into the first and/or second connection of the first chamber is measured.

15. The method according to claim 14, **characterised in that**
the integral over the physical and/or chemical property of the first fluid flowing into the first connection of the first chamber and the integral of the first fluid flowing out of the second connection of the first chamber are calculated before the reversal of the flow direction and
the integral over the physical and/or chemical property of the first fluid flowing into the second connection of the first chamber and the integral of the first fluid flowing out of the first connection of the first chamber are calculated after the reversal of the flow direction, wherein
the operation of the dialyser before the reversal of the flow direction with an equi-directional flow or with a counter-flow is determined on the basis of a comparison of the difference between the two integral values after the reversal of the flow direction with the difference between the two integral values before the reversal of the flow direction.

16. The method according to claim 15, **characterised in that** the quotient of the difference between the two integral values after the reversal of the flow direction and the difference between the two integral values before the reversal of the flow direction is calculated, wherein
it is concluded that there is an operation of the dialyser with an equi-directional flow before the reversal of the flow direction if the quotient is greater than 1 or it is concluded that there is an operation of the dialyser with a counter-flow before the reversal of the flow direction if the quotient is less than 1.

17. The method according to any one of claims 13 to 16, **characterised in that** the first fluid is dialysing fluid.

18. The method according to any one of claims 13 to 17, **characterised in that** the second fluid is blood.

19. The method according to any one of claims 13 to 18, **characterised in that** the physical and/or chemical property is the concentration of a substance in the fluid.

20. The method according to any one of claims 13 to 18, **characterised in that** the physical and/or chemical property is the temperature of the fluid.

21. The method according to any one of claims 13 to 19, **characterised in that** an optical and/or acoustic and/or tactile alarm is emitted if, before the reversal of the flow direction, an operation of the dialyser with an equi-directional flow is ascertained.

**Revendications**

1. Dispositif servant à identifier la direction de l'écoulement de fluide à travers un dialyseur (1), qui présente un premier compartiment (3), qui a un premier et un deuxième raccord (3A, 3B), et un deuxième compartiment (4), qui a un premier et un deuxième raccord (4A, 4B), le premier compartiment et le deuxième compartiment étant séparés l'un de l'autre par une membrane (2) semi-perméable,
comprenant
des moyens (16) servant à inverser le sens d'écoulement, lesquels sont réalisés de telle manière
que
le dialyseur
peut être commuté
entre un mode de fonctionnement en courant continu, dans lequel un premier liquide s'écoule dans le premier raccord du premier compartiment et le premier liquide s'écoule en dehors du deuxième raccord du premier compartiment, tandis que le deuxième liquide s'écoule dans le premier raccord du deuxième compartiment et que le deuxième liquide s'écoule en dehors du deuxième raccord du deuxième compartiment, et
un mode de fonctionnement en contre-courant, dans lequel le premier liquide s'écoule dans le deuxième raccord du premier compartiment et le premier liquide s'écoule en dehors du premier raccord du premier compartiment, tandis que le deuxième liquide s'écoule dans le premier raccord du deuxième compartiment et que le deuxième liquide s'écoule en dehors du deuxième raccord du deuxième compartiment,
ou
entre le mode de fonctionnement en contre-courant et le mode de fonctionnement en courant continu,
des moyens (5) servant à modifier une propriété physique et/ou chimique du liquide s'écoulant dans le premier ou le deuxième raccord du premier compartiment,
des moyens (17 ; 17B) servant à mesurer la propriété physique et/ou chimique du liquide s'écoulant en dehors du deuxième ou du premier raccord du premier compartiment, et
une unité de calcul et d'analyse (14) coopérant avec les moyens (17) servant à mesurer la propriété physique et/ou chimique, laquelle est réalisée de telle manière qu'on déduit sur la base de la modification, mesurée avant l'inversement du sens de l'écoulement ou après l'inversement du sens de l'écoulement, de la propriété physique et/ou chimique du premier liquide s'écoulant en dehors du deuxième ou du premier raccord du premier compartiment, laquelle est à lier à la modification de la propriété physique et/ou chimique du premier liquide s'écoulant dans le premier ou le deuxième raccord du premier compartiment, si le dialyseur a fonctionné en mode de fonctionnement en courant continu ou en mode de fonctionnement en contre-courant avant l'inversement du sens d'écoulement.

2. Dispositif selon la revendication 1, **caractérisé en ce que** les moyens (17 ; 17A) sont prévus pour mesurer la propriété physique et/ou chimique du liquide s'écoulant dans le premier ou le deuxième raccord du premier compartiment.

3. Dispositif selon la revendication 2, **caractérisé en ce que** l'unité de calcul et d'analyse (14) est réalisée de telle manière que
l'intégrale sur la propriété physique et/ou chimique du premier liquide s'écoulant dans le premier raccord du premier compartiment et l'intégrale du premier liquide s'écoulant en dehors du deuxième raccord du premier compartiment sont calculées avant l'inversement du sens de flux et
l'intégrale sur la propriété physique et/ou chimique du premier liquide s'écoulant dans le deuxième raccord du premier compartiment et l'intégrale du premier liquide s'écoulant en dehors du premier raccord du premier compartiment sont calculées après l'inversement du sens de flux
le fonctionnement du dialyseur étant déterminé avant l'inversement du sens de l'écoulement dans le courant continu ou dans le contre-courant sur la base d'une comparaison de la différence entre les deux valeurs d'intégrale après l'inversement du sens de l'écoulement à la différence des deux valeurs d'intégrale avant l'inversement du sens de l'écoulement.

4. Dispositif selon la revendication 3, **caractérisé en ce que** l'unité de calcul et d'analyse (14) est réalisée de telle manière que
le quotient de la différence entre les deux valeurs d'intégrale après l'inversement du sens de l'écoulement et de la différence des deux valeurs d'intégrale avant l'inversement du sens de l'écoulement est calculé,
un mode de fonctionnement du dialyseur avant l'inversement du sens d'écoulement dans le courant continu étant déduit lorsque le quotient est supérieur à 1 ou
un mode de fonctionnement du dialyseur avant l'inversement du sens d'écoulement dans le contre-courant étant déduit lorsque le quotient est inférieur à 1.

5. Dispositif selon l'une quelconque des revendications 1 à 4, **caractérisé en ce que** le premier liquide est un liquide de dialyse.

6. Dispositif selon l'une quelconque des revendications 1 à 5, **caractérisé en ce que** le deuxième liquide est du sang.

7. Dispositif selon l'une quelconque des revendications 1 à 6, **caractérisé en ce que** la propriété physique et/ou chimique est la concentration d'une matière dans le liquide, les moyens (17) servant à mesurer la propriété physique et/ou chimique étant des moyens servant à mesurer la concentration de matière.

8. Dispositif selon l'une quelconque des revendications 1 à 6, **caractérisé en ce que** la propriété physique et/ou chimique est la température du liquide, les moyens (17) servant à mesurer la propriété physique et/ou chimique étant des moyens servant à mesurer la température.

9. Dispositif selon l'une quelconque des revendications 1 à 8, **caractérisé en ce que** l'unité de calcul et d'analyse (14) coopère avec une unité d'alarme (18), qui émet une alarme optique et/ou acoustique et/ou tactile lorsque l'unité de calcul et d'analyse a constaté, avant l'inversement du sens de l'écoulement, un mode de fonctionnement du dialyseur en courant continu.

**10.** Dispositif selon l'une quelconque des revendications 1 à 9, **caractérisé en ce que** l'unité de calcul et d'analyse (14) génère un signal de commande pour une intervention dans la commande de machine lorsque l'unité de calcul et d'analyse a constaté, avant l'inversement du sens de l'écoulement, un mode de fonctionnement du dialyseur en courant continu.

**11.** Dispositif selon la revendication 10, **caractérisé en ce que** le signal de commande pour l'intervention dans la commande de machine est un signal de commande activant les moyens (16) servant à inverser le sens de flux.

**12.** Dispositif de traitement de sang extracorporel comprenant un circuit de sang (II) extracorporel, qui inclut le compartiment de sang (4) d'un dialyseur (1) divisé par une membrane (2) semi-perméable en le compartiment de sang et en un compartiment de liquide de dialyse (3), et comprenant un système de liquide de dialyse (I), qui inclut le compartiment de liquide de dialyse, **caractérisé en ce que** le dispositif de traitement de sang extracorporel présente un dispositif servant à identifier le sens de l'écoulement de liquide à travers un dialyseur selon l'une quelconque des revendications 1 à 11.

**13.** Procédé servant à identifier le sens de l'écoulement de liquide à travers un dialyseur, qui présente un premier compartiment, qui a un premier raccord et un deuxième raccord, et un deuxième compartiment, qui a un premier et un deuxième raccord, le premier compartiment et le deuxième compartiment étant séparés l'un de l'autre par une membrane semi-perméable,
une propriété physique et/ou chimique d'un premier liquide s'écoulant dans le premier raccord du premier compartiment étant modifiée, tandis qu'un deuxième liquide s'écoule dans le premier raccord du deuxième compartiment du dialyseur et en dehors du deuxième raccord du deuxième compartiment du dialyseur,
la propriété physique et/ou chimique du premier liquide s'écoulant en dehors du deuxième raccord du premier compartiment, laquelle est à lier à la modification de la propriété physique et/ou chimique du premier liquide s'écoulant dans le premier raccord du premier compartiment, étant mesurée,
le sens de l'écoulement étant inversé de telle manière que le premier liquide s'écoule dans le deuxième raccord du premier compartiment et que le premier liquide s'écoule en dehors du premier raccord du premier compartiment, et la question de savoir si le dialyseur a fonctionné avant l'inversement du sens d'écoulement dans le mode de fonctionnement en courant continu ou dans le mode de fonctionnement en contre-courant étant déduite sur la base de la modification, mesurée avant l'inversement du sens de l'écoulement et après l'inversement du sens de l'écoulement, de la propriété physique et/ou chimique du premier liquide s'écoulant en dehors du deuxième ou du premier raccord du premier compartiment, laquelle est à lier à la modification de la propriété physique et/ou chimique du premier liquide s'écoulant dans le premier ou le deuxième raccord du premier compartiment.

**14.** Procédé selon la revendication 13, **caractérisé en ce que** la propriété physique et/ou chimique du liquide s'écoulant dans le premier ou dans le deuxième raccord du premier compartiment est mesurée.

**15.** Procédé selon la revendication 14, **caractérisé en ce que** l'intégrale sur la propriété physique et/ou chimique du premier liquide s'écoulant dans le premier raccord du premier compartiment et l'intégrale du premier liquide s'écoulant en dehors du deuxième raccord du premier compartiment sont calculées avant l'inversement du sens de flux, et
en ce que l'intégrale sur la propriété physique et/ou chimique du premier liquide s'écoulant dans le deuxième raccord du premier compartiment et l'intégrale du premier liquide s'écoulant en dehors du premier raccord du premier compartiment sont calculées après l'inversement du sens de flux,
le mode de fonctionnement du dialyseur étant déterminé avant l'inversement du sens de l'écoulement en courant continu ou en contre-courant sur la base d'une comparaison de la différence entre les deux valeurs d'intégrale après l'inversement du sens de l'écoulement avec la différence des deux valeurs d'intégrale avant l'inversement du sens de l'écoulement.

**16.** Procédé selon la revendication 15, **caractérisé en ce que** le quotient de la différence entre les deux valeurs d'intégrale après l'inversement du sens de l'écoulement et de la différence entre les deux valeurs d'intégrale avant l'inversement du sens de l'écoulement est calculé,
un mode de fonctionnement du dialyseur avant l'inversement du sens de l'écoulement dans le courant continu étant déduit lorsque le quotient est supérieur à 1 ou
un mode de fonctionnement du dialyseur avant l'inversement du sens de l'écoulement en contre-courant étant déduit lorsque le quotient est inférieur à 1.

**17.** Procédé selon l'une quelconque des revendications 13 à 16, **caractérisé en ce que** le premier liquide est un liquide de dialyse.

**18.** Procédé selon l'une quelconque des revendications 13 à 17, **caractérisé en ce que** le deuxième liquide est du sang.

**19.** Procédé selon l'une quelconque des revendications 13 à 18, **caractérisé en ce que** la propriété physique et/ou chimique est la concentration d'une substance dans le liquide.

**20.** Procédé selon l'une quelconque des revendications 13 à 18, **caractérisé en ce que** la propriété physique et/ou chimique est la température du liquide.

**21.** Procédé selon l'une quelconque des revendications 13 à 19, **caractérisé en ce qu'**une alarme optique et/ou acoustique et/ou tactile est émise, lorsqu'un mode de fonctionnement du dialyseur en courant continu est constaté avant l'inversement du sens de l'écoulement.

Fig. 1

EP 2 598 186 B1

Fig. 2

EP 2 598 186 B1

Fig. 3

EP 2 598 186 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2276566 A1

- WO 0002604 A1